# EUROPEAN PATENT APPLICATION

(11) **EP 4 677 998 A1**
(43) Date of publication of application: **14.01.2026**
(21) Application number: 24306173.6
(22) Date of filing: 11.07.2024
(51) Int. Cl.: A01H 1/00, C07K 14/415

(54) **A METHOD OF PRODUCING A PLANT WITH AN INCREASED DENSITY OF GLANDULAR TRICHOMES AND/OR AN INCREASED PRODUCTION OF METABOLITES**

(71) Applicant: Institut National de la Recherche pour l'Agriculture, l'Alimentation et l'Environnement, 75007 Paris (FR); Symrise AG, 37603 Holzminden Niedersachsen (DE)
(72) Inventor: BOUALEM, Adnane, 91405 Orsay (FR); BENDAHMANE, Abdelhafid, 91405 Orsay (FR); JOIN, Benoit, 37603 Holzminden (DE); Panten, Johannes, 37671 Höxter (DE)
(74) Representative: Gevers & Orès

(57) **Abstract**

The present invention relates to a method of producing a plant with an increased density of glandular trichomes and/or with an increased production of metabolite(s) comprising
a step of introducing at least one genetic alteration that increases activity of PRODERMAL FACTOR2 (PDF2) of said plant, wherein PDF2 preferably has an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

## Description

### FIELD OF THE INVENTION

The present invention relates to a method of producing and/or identifying a plant with an increased density of glandular trichomes and/or an increased production of at least one metabolite. The present invention relates to a genetically engineered plant with an increased density of glandular trichomes and/or an increased production of at least one metabolite.

### BACKGROUND

The plant epidermis is a single layer of cells that covers plant organs and directly in contact with the environment. In addition to its roles as barrier in the interaction of plants with their fluctuating biotic and abiotic environment, it plays central roles in growth and development and the acquisition of nutrients. This diversity of epidermal functions requires a range of epidermal cell types, from root hairs, stomata and different sorts of outgrowths in the aerial parts, known as trichomes (Lolle and Pruitt, 1999; Glover, 2000).

Trichomes are specialized organs that result from epidermal outgrowths. There is two distinct classes of trichomes, non-glandular and glandular trichomes.

Non-glandular trichomes (non-GTs) are mostly unicellular and can be either unbranched, or have two to five branches (Ishida *et al.,* 2008). They are present on most angiosperms and on some gymnosperms and bryophytes. Their suggested roles include transpiration control, alleviating heating and damages caused by UV radiations and protection of the plant body from insects (Karabourniotis *et al.,* 1992; Yan *et al.,* 2012; Figueiredo *et al.,* 2013).

In contrast to non-GTs, glandular trichomes (GTs) are generally multicellular, consisting of differentiated basal, stalk and secretory gland cells (Chalvin *et al.,* 2020; Lange, 2015). They are widely present in vascular plants and they usually cover plant surfaces like leaves, flowers and stems. They are present in more than 30% of the vascular plants and usually cover plant leaves, flowers and stems (Fahn *et al.,* 2000). In angiosperms, the most notable function of GTs is their role in plant defense against pests. GTs can be viewed as a combination of structural and chemical defenses against insect pests and herbivory. The most singular feature of GTs is their unique capacity to synthesize, store and sometimes secrete a wide variety of structurally and biosynthetically diverse metabolites, including polysaccharides, organic acids, proteins, terpenoids, alkaloids and polyphenols. (Huchelmann *et al.,* 2017). The metabolic activity of the GTs is often dominated by the production of terpenoids and flavonoids, two of the most diverse and economically important classes of natural metabolites. These specialized metabolites are assembled from organic building blocks synthetized by different pathways. Some are known to be involved in the attraction of pollinators as well as defense against important insect pests.

Broad interest in these epidermal "biofactories" is heightened by the fact that many GTs-derived specialized metabolites have become commercially important as natural pesticides, but also have found uses as fragrances, food additives and drugs.

The ability to modulate the density and productivity of glandular trichomes would thus be of great biotechnological interest. This requires the identification and characterization of the genes initiating, regulating, and driving the development of such glandular structures (A. Huchelmann *et al.,* 2017). How non-GTs are initiated and develop have been extensively investigated in *Arabidopsis thaliana* (Pattanaik *et al.,* 2014, ). However, data on how GTs develop are scattered. This mainly because the model plant species, *A. thaliana,* lacks GTs and the extrapolation of the knowledge gathered on non-GTs to GTs was unsuccessful (Serna and Martin, 2006). Several lines of evidence indicate that glandular (and in general multicellular) trichome formation is probably controlled by a different network than the one controlling nonglandular trichome formation in *Arabidopsis thaliana* (Chalvin *et al.,* 2020). Several transcription factors involved in glandular trichome initiation have been identified both in *Solanum lycopersicum* and *Artemisia annua* (Chalvin *et al.,* 2020). In consequence, very little is known on how epidermal cells differentiate to produce GTs and to date, these discoveries remained insufficient to improve the production of metabolites with high industrial or pharmacological value from glandular trichomes.

### SUMMARY OF THE INVENTION

The Inventors have found a new transcription factor involved in the development of glandular trichome : the PROTODERMAL FACTOR2 (PDF2). Indeed, the inventors have shown that the lack of expression of PDF2 results in a significant decrease of the glandular trichome density. Moreover, they have identified gain of function mutations in the *PDF2* gene that leads to plants with an increased density of glandular trichomes resulting in an increased production of metabolites of interest.

Therefore, in a first aspect, the present invention relates to a method of producing a plant with an increased density of glandular trichomes comprising:
a step of introducing at least one genetic alteration that increases activity of PRODERMAL FACTOR2 (PDF2) of said plant, wherein PDF2 preferably has an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39 or an amino acid sequence having at least 70%, preferably at least 75 %, moreover preferably at least 80 %, moreover preferably at least 85 %, moreover preferred at least 90 % and especially preferred at least 99 % identity with SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

In a second aspect, the present invention relates to a method of producing a plant with an increased production of at least one metabolite comprising:
a step of introducing at least one genetic alteration that increases the activity of PDF2 of said plant, wherein PDF2 preferably has an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39 or an amino acid sequence having at least 70%, preferably at least 75 %, moreover preferably at least 80 %, moreover preferably at least 85 %, moreover preferred at least 90 % and especially preferred at least 99 % identity with SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

In one embodiment of the first and second aspect of the invention, the at least one genetic alteration that increases activity of PDF2 comprises at least one mutation in *PDF2* gene encoding PDF2.

In another embodiment of the first and second aspect of the invention, the at least one genetic alteration that increases activity of PDF2 comprises the overexpression of PDF2, preferably the overexpression of PDF2 due to the introduction of genetic alteration(s) in the promoter of *PDF2.*

In a third aspect, the present invention relates to a method for identifying a plant having an increased density of glandular trichomes and/or an increased production of at least one metabolite comprising the step of the detecting in the plant at least one genetic alteration that increases activity of PDF2, preferably at least one mutation in *PDF2* gene that increases the activity PDF2.The present invention also relates to a kit for identifying a plant having an increased density of glandular trichomes and/or an increased production of at least one metabolite, wherein said kit comprises an oligonucleotide primer pair for amplifying a region of *PDF2* gene, preferably of the region encoding the START domain, wherein the at least one mutation in *PDF2* gene that increases activity of PDF2 is detected with the oligonucleotides. In a fourth aspect of the invention, the present invention relates to a genetically engineered plant obtained or obtainable by the methods according to the first and/or second aspect of the invention, wherein said plant has an increased density of glandular trichomes and/or an increased production of at least one metabolite compared to a control plant; as well as the use of the plant of the invention for producing at least one metabolite.

### DETAILED DESCRIPTION

### Definitions

As used herein, the term sequence "identity" has art-recognized meanings and the percentage of sequence identity between two nucleic acid or polypeptide molecules or regions can be calculated using published techniques. The percentage of sequence identity between two amino acid sequences is defined as the percentage of amino acid residues in that are identical between these sequences.

Methods of alignment of sequence for comparison are well-known in the art. Optimal alignment of sequences for comparison can be conducted by the local homology algorithm of Smith *et al.* (1981), by the homology alignment algorithm of Needleman et al. (J. MoL Biol. 48:443 (1970), by the search for similarity method of Person et al. (Proc. Natl. Acad. Sci. USA, 85, 2444 (1988)), by computerized implementations of these algorithms (GAP, BESTFIT, FASTA, and TFASTA in the Wisconsin Genetics Software Package, Genetics Computer Group, 575 Science Dr., Madison, Wl), or by manual alignment and visual inspection. Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wisconsin, USA). Alignments using these programs can be performed using the default parameters. The CLUSTAL program is well described by Higgins et al., Gene, 73:237 (1988); Higgins et al., CABIOS. 5:157 (1989); Corp et et al., Nucl. Acids Res.. 16:1088 (1988); Huang et al., CABIOS. 8:155 (1992); and Pearson et al., Methods MoL Biol. 24:307 (1994). The ALIGN program is based on the algorithm of Myers and Miller, LABIOS. 4: 11 (1988). The BLAST programs of Altschul et al. (J. MoL Biol., 215:403 (1990)) are based on the algorithm of Karlin and Altschul (PNAS USA. 90:5873 (1993)).

A "gene" is a defined region that is located within a genome and comprises a coding nucleic acid sequence and typically also comprises other, primarily regulatory, nucleic acids responsible for the control of the expression, that is to say the transcription and translation, of the coding portion. A gene may also comprise other 5' and 3' untranslated sequences and termination sequences. Further elements that may be present are, for example, introns.

A "nucleic acid molecule" or "nucleic acid sequence" is a segment of single- or doublestranded DNA or RNA that can be isolated from any source.

The terms "protein," "peptide" and "polypeptide" are used interchangeably herein.

As used herein, "codon optimized" sequence means the nucleotide sequence of a recombinant, transgenic, or synthetic polynucleotide wherein the codons are chosen to reflect the particular codon bias that a host cell may have. This is done in such a way so as to preserve the amino acid sequence of the polypeptide encoded by the codon optimized polynucleotide.

Nucleotides are indicated by their bases by the following standard abbreviations: adenine (A), cytosine (C), thymine (T), and guanine (G). Amino acids are likewise indicated by the following standard abbreviations: alanine (Ala; A), arginine (Arg; R), asparagine (Asn; N), aspartic acid (Asp; D), cysteine (Cys; C), glutamine (Gin; Q), glutamic acid (Glu; E), glycine (Gly; G), histidine (His; H), isoleucine (He; 1), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V).

Unless otherwise specified, a "plant" is any plant able to produce glandular trichomes at any stage of development, particularly a plant seed.

In the context of the invention and unless otherwise specified, an increased activity of PDF2 means an increased activity of PDF2 as compared to a control plant. Specifically, in the context of the invention, "activity" of PDF2 refers to the activity of PDF2 in relation to the glandular trichome development.

As used therein, a control plant is of the same genetic background and is growing in the same environment as the described plant, but it does not comprise the at least one genetic alteration, while the described plant does comprise the at least one genetic alteration according to the invention.

Preferably, the method according to the invention is not an essentially biological process.

Genetic alteration may be carried out by any genetic engineering techniques.

Genetic engineering techniques are well known by the person skilled in the art and include mutagenesis, such as, ionizing radiation (X-ray, Gamma rays and neutrons); chemical mutagenesis; genome editing with site-directed nucleases (SDNs), e.g., using clustered regularly interspaced short palindromic repeat (CR!SPR)-directed nucleases, transcription activator-like effector nuclease (TALENs), zinc-finger nucleases (ZFNs) or meganucleases; and targeting induced local lesions in genomes (TILLING).

"TILLING" is a method of reverse genetics which is based on the ability of an endonuclease to detect mismatches in a double strand of DNA and to create a DNA cleavage at the unpaired bases (MC Callum et al., 2000, Plant Physiology, Vol. 123: 439-442). TILLING could be also based on direct sequencing of amplicons of mutant lines or full sequencing of mutant lines to identify individual plants harboring induced mutations. This technique makes it possible to detect unique mutation points generated by the exposure of plants to a mutagenic chemical compound (http://ips2.u-psud.fr/en/platforms/epitrans-epigenomic-translational-biology/applications/mutation-screening.html). TILLING technique thus allows the identification of a series of alleles of a given gene and is particularly well suited to the implementation of methods of high throughput screenings for allowing the selection, in the target genes of interest, mutations induced by chemical mutagenesis, like EMS chemical exposition.

The chemical mutagenesis may be realized by exposing the plant cells, the protoplasts or the seeds to the EMS (ethyl methanesulfonate, see Koornbeef et al., 1982, Mutat Res, Vol. 93: 109-123), sodium azide or methylnitrourea mutagenic agents.

Mutagenesis can also be carried by a combination of TILLING and gene editing technics as described in Jacob P. et al., 2018 (Translational Research: Exploring and Creating Genetic Diversity Trends in Plant Sciences 23(1):42-52).

CRISPR is based on a bacterial immune system against invading bacteriophages in which a complex of 2 small RNAs, the CR!SPR-RNA (crRNA) and the trans-activating crRNA (tracrRNA) directs a nuclease (Cas9) to a specific DNA sequence complementary to the crRNA.

In the context of the invention, the increased activity may results from an overexpression and/or from an increased intrinsic activity of the gene or of the gene product. Methods for increasing expression or intrinsic activity of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. If for example increased expression or activity of an endogenous gene is desired, isolated nucleic acids which serve as promoter or enhancer elements may be introduced in an appropriate position (typically upstream) of a non-heterologous form of a polynucleotide so as to upregulate expression of a PDF2 nucleic acid or variant thereof. As used therein, unless otherwise specified, metabolite(s) (also called specialized or secondary metabolite(s)) means metabolite(s) produced by glandular trichome.

### Methods

In a first aspect, the present invention relates to a method of producing a plant with an increased density of glandular trichomes comprising:
a step of introducing at least one genetic alteration PRODERMAL FACTOR2 (PDF2)that increases activity of PRODERMAL FACTOR2 (PDF2), wherein PDF2 preferably has an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39 or an amino acid sequence having at least 70%, preferably at least 75 %, moreover preferably at least 80 %, moreover preferably at least 85 %, moreover preferred at least 90 % and especially preferred at least 99 % identity with SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

In a preferred embodiment, the present invention relates to a method of producing a plant with an increased density of glandular trichomes comprising:
a step of introducing at least one genetic alteration that increases activity of PRODERMAL FACTOR2 (PDF2) of said plant, PDF2 having the amino acid sequence SEQ ID NO: 1 or an amino acid sequence having at least 70%, at least 75%, at least 80%, at least 85%, at least 90%, at least 95%, at least 96%, at least 97%, at least 98% or at least 99% identity with SEQ ID NO: 1.

The amino acid sequences SEQ ID NO: 1 corresponds to the PDF2 protein of *Salvia sclarea* in which a mutation has been shown by the inventors to increase the glandular trichome density and the metabolites production as detailed in the Examples.

Advantageously, said mutation results in an increased density of glandular trichomes compared to a control plant.

PROTODERMAL FACTOR2 (*PDF2*) is a class IV homeodomain-leucine zipper (HD-ZIP IV) protein. HD-ZIP IV proteins represent plant-specific transcription factors, most of which are expressed predominantly in the epidermis (M. Nakamura et al., (2006), Characterization of the Class IV Homeodomain-Leucine Zipper Gene Family in Arabidopsis, Plant Physiology, Volume 141, Issue 4, Pages 1363-1375). HD-ZIP IV families of proteins are characterized by an amino-terminal HD-ZIP region followed by a region similar to the mammalian StAR-related lipid-transfer (START) domain (Ponting CP, et al. (1999) START: a lipid-binding domain in StAR, HD-ZIP and signalling proteins. Trends Biochem Sci 24 : 130-132).

Examples of various plant species and their corresponding SEQ ID NO for PDF2 and START domain are given in the following table 1.

| **Plant species** | | **SEQ ID NO** |
|---|---|---|
| *Salvia sclarea* (clary sage) | PDF2 | 1 |
| | START | 16 |
| *Salvia sclarea* mutant A347S | PDF2 | 36 |
| | START | 37 |
| *Salvia sclarea* mutant L319M | PDF2 | 48 |
| | START | 49 |
| *Cannabis sativa* (cannabis) | PDF2 | 2 |
| | START | 17 |
| *Artemisia annua* (sweet wormwood) | PDF2 | 3 |
| | START | 18 |
| *Salvia splendens* (scarlet sage) | PDF2 | 4 |
| | START | 19 |
| *Salvia hispanica* (chia) | PDF2 | 5 |
| | START | 20 |
| *Perilla frutescens* (Korean perilla) | PDF2 | 6 |
| | START | 21 |
| *Thymus quinquecostatus* | PDF2 | 7 |
| | START | 22 |
| *Mentha longifolia* (horse mint) | PDF2 | 8 |
| | START | 23 |
| *Solanum lycopersicum* (tomato) | PDF2 | 9 |
| | START | 24 |
| *Solanum tuberosum* (potato) | PDF2 | 10 |
| | START | 25 |
| *Capsicum annuum* (red pepper) | PDF2 | 11 |
| | START | 26 |
| | | |
| *Cucumis melo* (melon) | PDF2 | 12 |
| | START | 27 |
| *Cucumis sativus* (cucumber) | PDF2 | 13 |
| | START | 28 |
| *Gossypium hirsutum* (upland cotton) | PDF2 | 14 |
| | START | 29 |
| *Nicotiana tabacum* (tobacco) | PDF2 | 15 |
| | START | 30 |
| *Salvia rosmarinus* | PDF2 | 38 |
| | START | 40 |
| *Salvia officinalis* | PDF2 | 39 |
| | START | 41 |

In an embodiment, PDF2 has the amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

In a preferred embodiment, PDF2 has the amino acid sequence selected from the group consisting of SEQ ID NO: 1, SEQ ID NO: 4, SEQ ID NO: 5, SEQ ID NO: 38 and SEQ ID NO: 39. In a more preferred embodiment, the PDF2 protein has the amino acid sequence SEQ ID NO: 1.

In an embodiment, the at least one genetic alteration that increases activity of PDF2 comprises, preferably consists of, at least one mutation in the gene encoding PDF2.

Thus, an aspect of the invention relates to a method of producing a plant with an increased density of glandular trichomes comprising:
a step of introducing at least one mutation in a *PDF2* gene of said plant, the *PDF2* gene encoding a PDF2 protein, wherein PDF2 preferably has an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39 or an amino acid sequence having at least 70%, preferably at least 75 %, moreover preferably at least 80 %, moreover preferably at least 85 % and preferred at least 90 %, moreover preferably at least 95 % and especially preferably at least 99 % identity with SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

As mentioned above, the amino acid sequence SEQ ID NO: 1 corresponds to the PDF2 protein of *Salvia sclarea.* The PDF2 gene of *Salvia sclarea* has the genomic sequence SEQ ID NO: 32 and has the coding DNA sequence SEQ ID NO: 33.

Thus, according to a preferred embodiment of the invention, at least one genetic alteration is introduced in the nucleotide sequence SEQ ID NO: 32.

In a preferred embodiment, the at least one mutation introduced in the *PDF2* gene comprises a mutation in the region encoding the *StAR-related lipid-transfer* (*START*) domain of PDF2, the START domain having an amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 40 and SEQ ID NO: 41 or an amino acid sequence having at least 70%, %, preferably at least 75 %, moreover preferably at least 80 %, moreover preferably at least 85 % and especially preferred at least 90 %, at least 95%, at least 96%, at least 97% identity with an amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 40 and SEQ ID NO: 41.

In a preferred embodiment, the at least one mutation introduced in the *PDF2* gene comprises a mutation in a region of the *PDF2* gene encoding a START domain, the START domain having an amino acid sequence SEQ ID NO: 16 or an amino acid sequence having at least 70%, at least 75%, at least 80%, at least 85%, at least 90%, at least 95%, at least 96%, at least 97%, at least 98% or at least 99% identity with SEQ ID NO: 16.

In a more preferred embodiment, the at least one mutation introduced in the *PDF2* gene consists of a mutation in a region of the *PDF2* gene encoding a START domain, the START domain having an amino acid sequence SEQ ID NO: 16 or an amino acid sequence having at least 70%, at least 75%, at least 80%, at least 85%, at least 90%, at least 95%, at least 96%, at least 97%, at least 98% or at least 99% identity with SEQ ID NO: 16.

The amino acid sequence SEQ ID NO: 16 corresponds to the START domain of the PDF2 protein of *Salvia sclarea.*

The table 1 above shows examples of amino acid sequences of START domain.

Thus, in an embodiment, the START domain has the amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 40 and SEQ ID NO: 41.

In a preferred embodiment, the START domain has the amino acid sequence selected from the group consisting of SEQ ID NO: 16, SEQ ID NO: 19, SEQ ID NO: 20, SEQ ID NO: 40 and SEQ ID NO: 41. In a more preferred embodiment, the START domain has the amino acid sequence SEQ ID NO: 16.

A comparison of the different amino acid sequences of the START domain set forth in Table 1 shows that this sequence is highly conserved among the different plants.

The inventors have identified mutations resulting in an increased density of glandular trichomes and in an increased production of metabolites in the central region of the START domain. Thus, in an embodiment, the at least one mutation comprises a mutation in the nucleic acid sequence that encodes the amino acids which corresponds to the amino acids 58-117, preferably 71-113, more preferably 79-113 of the START domain having SEQ ID NO: 16.ln an embodiment, the at least one mutation comprises a mutation in the nucleic acid sequence that encodes the amino acids which corresponds to a subsequence of the START domain has the amino acid sequence SEQ ID NO: 31, corresponding the amino-acids 58-117 of the START domain having SEQ ID NO: 16 : X₁X₂X₃GX₄X₅X₆EASRX₇X₈X₉VVIX₁₀X₁₁X₁₂X₁₃X₁₄LVEILMDX₁₅X₁₆QWX₁₇X₁₈X₁₉FX₂₀X₂₁X₂₂VSX₂₃X₂₄X₂₅ X₂₆LX₂₇X₂₈LSTGVAGNX₂₉NGA , with X₁= K or Q, X₂= P or S, X₃= L or I or S, X₄= L or M or F, X₅=K or R or I or T, X₆= SorT or C, X₇=E or Q, X₈= SorT or L, X₉= N or A or S, X₁₀= I or M, X₁₁= N or D, X₁₂=Y or H, X₁₃= Lor T or I or V, X₁₄= H or N or S or T, X₁₅= V or T, X₁₆=S or N or T, X₁₇=T or S, X₁₈= N or T or S, X₁₉= F or I or M or V or L, X₂₀= T or C or A or S, X₂₁= G or S, X₂₂= I or L, X₂₃= K or R, X₂₄= S or A, X₂₅= T or M or L or V or A, X₂₆= Lor T, X₂₇= E or D, X₂₈=V or I and X₂₉=Y or H.

In an embodiment, the at least one mutation comprises a mutation in the nucleic acid sequence that encodes the amino acids which corresponds to a consensus sequence of the START domain of the *Lamiaceae* having SEQ ID NO: 55 : X₁X₂ADKPVIIELAVAAMEELX₃RMX₄GQX₅GX₆PLWX₇X₈X₉X₁₀X₁₁X₁₂X₁₃X₁₄EX₁₅X₁₆X₁₇X₁₈X₁₉X₂₀YX₂₁X₂₂X₂₃FPRGI GPKPLGLX₂₄SEASRX₂₅X₂₆X₂₇VVIMNHIX₂₈LVEILMDX₂₉X₃₀QWSX₃₁X₃₂FX₃₃SIVSRX₃₄ X₃₅TX₃₆ EVLSTGVAGNX₃₇ NGAX₃₈QVMTAEFQVPSPLVPTREX₃₉YFVRYCKQHX₄₀DGX₄₁WAVVDVSLDNLRPX₄₂X₄₃ISX₄₄CRRRPSGCX₄₅IQE LPNGYSKVTWX₄₆EHVEX₄₇DDRX₄₈VHSIYKX₄₉LVN X₅₀GLAFGAX₅₁RWVAX₅₂LDRQCERLAS, with X₁ = S or T, X₂ = D or E, X₃ = I or L or V, X₄ = A or G, X₅ = S or T or A, X₆ = E or D, X₇ = M or I or L, X₈ = P or Q, X₉ = S or T, X₁₀ = E or D, X₁₁ = N or NOTHING, X₁₂ = S or G or NOTHING, X₁₃ = A or G or L or S or NOTHING, X₁₄ = A or G or V, X₁₅ = L or I or M or V, X₁₆ = L or F, X₁₇ = S or V or C, X₁₈ = E or D, X₁₉ = E or D, X₂₀ = E or D, X₂₁ = G or A or V, X₂₂ = R or T, X₂₃ = I or T, X₂₄ = K or I or R, X₂₅ = E or Q, X₂₆ = S or L or T, X₂₇ = A or S, X₂₈ = K or N or L, X₂₉ = V or T, X₃₀ = N or T, X₃₁ = S or N, X₃₂ = V or M, X₃₃ = A or S, X₃₄ = S or A, X₃₅ = V or M, X₃₆ = L or T, X₃₇ = Y or H, X₃₈ = L or F, X₃₉ = N or I, X₄₀ = T or H, X₄₁ = T or S, X₄₂ = T or A or S, X₄₃ = S or N, X₄₄ = R or K, X₄₅ = L or V, X₄₆ = V or I, X₄₇ = V or I, X₄₈ = A or T, X₄₉ = P or A, X₅₀ = T or A or S, X₅₁ = K or R, X₅₂ = T or I.

In accordance with the present invention, the term " at a position which corresponds to the position XY of SEQ ID NO:Z", wherein X is the amino acid being at position Y of the reference sequence SEQ ID NO: Z, does not only include the respective position in the SEQ ID NO referred to afterwards but also includes any sequence encoding a variant of the reference SEQ ID NO: Z, where, after alignment with the reference SEQ ID NO: Z, the respective position might have a different number but corresponds to that indicated for the reference SEQ ID NO: Z.

The mutations resulting in an increased density of glandular trichomes which has been identified by the inventors are:
- a substitution of the alanine (A) at position 347 of SEQ ID NO: 1 with a serine (S) and
- a substitution of the leucine (L) at position 319 of SEQ ID NO: 1 with a methionine (M).

The amino acids 315-321 (LVEILMD SEQ ID NO: 53) and 342-349 (LSTGVAGN SEQ ID NO: 54) of PDF2 having SEQ ID NO: 1 where the mutations gain of function have been found by the inventors are highly conserved among the various plant families.

Thus, in an embodiment, the at least one mutation comprises or consists in a mutation in the nucleic acid sequence that encodes the amino acids which corresponds to the amino acids 315-321 and/or 342-349 of PDF2 having SEQ ID NO: 1.

In the context of the invention, an alternative substitution to a substitution from a wild type amino acid with a mutant amino acid may be done with amino acids of which side chains has similar chemical properties of the mutant amino acid e.g. 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; 7) sulfur-containing side chains: cysteine and methionine; and 8) cyclic: proline.

In an embodiment, the at least one mutation comprises or consists in:
- a mutation that results in a substitution with a M or C preferably a M, at a position which corresponds to the position selected from the group consisting of L315, I318 and L319 of PDF2 having the amino acid sequence SEQ ID NO: 1
   and/or
- a mutation that results in a substitution with a S, T, N or Q, preferably a S, at a position which corresponds to the position selected from the group consisting of L342, G345, A347, and G348 of PDF2 having the amino acid sequence SEQ ID NO: 1.

In an embodiment, the at least one mutation comprises or consists in:
- a mutation that results in a substitution with a S, T, N or Q, preferably a S, at a position which corresponds to the position A347 of PDF2 having the amino acid sequence SEQ ID NO: 1 and/or
- a mutation that results in a substitution with a M or C, preferably a M, at a position which corresponds to the position L319 of PDF2 having the amino acid sequence SEQ ID NO: 1.

In an embodiment, the at least one mutation comprises or consists in a mutation that results in a substitution, preferably with a S, T, N or Q, more preferably with a S, at position which corresponds to the position A347 of the amino acid sequence SEQ ID NO: 1.

The position A347 of the amino acid sequence SEQ ID NO: 1 corresponds to: position 371 of the amino acid sequence SEQ ID NO: 2
position 349 of the amino acid sequence SEQ ID NO: 3,
position 349 of the amino acid sequence SEQ ID NO: 4,
position 345 of the amino acid sequence SEQ ID NO: 5,
position 334 of the amino acid sequence SEQ ID NO: 6,
position 348 of the amino acid sequence SEQ ID NO: 7,
position 349 of the amino acid sequence SEQ ID NO: 8,
position 339 of the amino acid sequence SEQ ID NO: 9,
position 354 of the amino acid sequence SEQ ID NO: 10,
position 349 of the amino acid sequence SEQ ID NO: 11,
position 351 of the amino acid sequence SEQ ID NO: 12,
position 351 of the amino acid sequence SEQ ID NO: 13,
position 347 of the amino acid sequence SEQ ID NO: 14,
position 349 of the amino acid sequence SEQ ID NO: 15,
position 346 of the amino acid sequence SEQ ID NO: 38 and
position 347 of the amino acid sequence SEQ ID NO: 39.

In an embodiment, the at least one mutation comprises or consists in a mutation that results in a substitution with a S at a position which corresponds to the position A347 of PDF2 having the amino acid sequence SEQ ID NO: 1.

In an embodiment, the at least one mutation comprises or consists in a mutation that results in a substitution A347S in PDF2 having the amino acid sequence SEQ ID NO: 1.

The A347S mutation in PDF2 corresponds to a substitution of A with a S at position 111 of the START domain having the amino acid sequence SEQ ID NO: 16.

In an embodiment, the at least one mutation comprises or consists in a mutation that results in a substitution at position which corresponds to the position 111 of START domain of PDF2 having the amino acid sequence SEQ ID NO: 16.

In an embodiment, the at least one mutation comprises or consists in a mutation that results in a substitution with a S or a T, preferably with a S at a position which corresponds to the position 111 of START domain of PDF2 having the amino acid sequence SEQ ID NO: 16.

In an embodiment, the at least one mutation comprises or consists in a mutation that results in a substitution, preferably with a S or a T, preferably with a S at the position 55 of the sub sequence of the START domain of the PDF2 protein having the amino acid sequence SEQ ID NO: 31.

In an embodiment, the at least one mutation comprises or consists in a mutation that results in a substitution with a S or a T , preferably with a S, in START domain of PDF2 having the amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 30, SEQ ID NO: 40 and SEQ ID NO: 41, at a position which corresponds to the position 111 of the START domain having the amino acid sequence SEQ ID NO: 1.

In an embodiment, the at least one mutation comprises or consists in a mutation selected from the group consisting of:
- a substitution with a nucleotide T at a position which corresponds to position 3 209 of SEQ ID NO: 32,
- a substitution with a nucleotide A at a position which corresponds to position 3 209 of SEQ ID NO: 32,
- a substitution with a nucleotide C at a position which corresponds to position 3 209 of SEQ ID NO: 32 and
- a substitution with a nucleotide A at a position which corresponds to position 3 209 of SEQ ID NO: 32 and with a T or C at a position which corresponds to position 3 211 of SEQ ID NO: 32.

In an embodiment, the mutated *PDF2* gene has the nucleic acid sequence SEQ ID NO: 34 (which corresponds to the genomic sequence SEQ ID NO: 32 mutated with a substitution from G to T at position 3 209).

In an embodiment, the mutated *PDF2* gene has the nucleic acid sequence SEQ ID NO: 35 (which corresponds to the coding DNA sequence SEQ ID NO: 33 mutated with a substitution from G to T at a position which corresponds to position 3 209 of SEQ ID NO: 32).

In an embodiment, the at least one mutation comprises or consists in a mutation that results in a substitution, preferably with a M or C, more preferably with a M, at position which corresponds to the position L319 of the amino acid sequence SEQ ID NO: 1.

The position L319 of the amino acid sequence SEQ ID NO: 1 corresponds to:
position 341 of the amino acid sequence SEQ ID NO: 2
position 321 of the amino acid sequence SEQ ID NO: 3,
position 321 of the amino acid sequence SEQ ID NO: 4,
position 317 of the amino acid sequence SEQ ID NO: 5,
position 306 of the amino acid sequence SEQ ID NO: 6,
position 320 of the amino acid sequence SEQ ID NO: 7,
position 321 of the amino acid sequence SEQ ID NO: 8,
position 311 of the amino acid sequence SEQ ID NO: 9,
position 326 of the amino acid sequence SEQ ID NO: 10,
position 321 of the amino acid sequence SEQ ID NO: 11,
position 323 of the amino acid sequence SEQ ID NO: 12,
position 323 of the amino acid sequence SEQ ID NO: 13,
position 319 of the amino acid sequence SEQ ID NO: 14,
position 321 of the amino acid sequence SEQ ID NO: 15,
position 318 of the amino acid sequence SEQ ID NO: 38 and
position 319 of the amino acid sequence SEQ ID NO: 39.

In an embodiment, the at least one mutation comprises or consists in a mutation that results in a substitution with a M at a position which corresponds to the position L319 of the PDF2 protein having the amino acid sequence SEQ ID NO: 1.

In an embodiment, the at least one mutation comprises or consists in a mutation that results in a substitution L319M in PDF2 having the amino acid sequence SEQ ID NO: 1.

The L319M mutation corresponds to a substitution of L with a M at position 83 of the START domain having the amino acid sequence SEQ ID NO: 16.

In an embodiment, the at least one mutation comprises or consists in a mutation that results in a substitution at position which corresponds to the position 83 of the START domain of PDF2 having the amino acid sequence SEQ ID NO: 16.

In an embodiment, the at least one mutation comprises or consists in a mutation that results in a substitution with a M or C preferably with a M at a position which corresponds to the position 83 of the START domain of PDF2 having the amino acid sequence SEQ ID NO: 16.

In an embodiment, the at least one mutation comprises or consists in a mutation that results in a substitution, with a M or C, preferably with a M, at the position 83 of the START domain of PDF2 having the amino acid sequence SEQ ID NO: 31.

In an embodiment, the at least one mutation comprises or consists in a mutation that results in a substitution with a M or C, preferably with a M, in START domain having the amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 30, SEQ ID NO: 40 and SEQ ID NO: 41, at a position which corresponds to the position 83 of the START domain having the amino acid sequence SEQ ID NO: 1.

In an embodiment, the at least one mutation comprises or consists in a mutation selected from the group consisting of:
- a substitution with a nucleotide A at a position which corresponds to position 3 042 of SEQ ID NO: 32,
- a substitution with a nucleotide G at a position which corresponds to position 3 043 of SEQ ID NO: 32 and with a nucleotide T at a position which corresponds to position 3 044 of SEQ ID NO: 32,
- a substitution with a nucleotide G at a position which corresponds to position 3 043 of SEQ ID NO: 32 and with a nucleotide C at a position which corresponds to position 3 044 of SEQ ID NO: 32.

In an embodiment, the mutated *PDF2* gene has the nucleic acid sequence SEQ ID NO: 46 (which corresponds to the genomic sequence SEQ ID NO: 32 mutated with a substitution from T to A at position 3 042).

In an embodiment, the mutated *PDF2* gene has the nucleic acid sequence SEQ ID NO: 47 (which corresponds to the coding DNA sequence SEQ ID NO: 33 mutated with a substitution from T to A at a position which corresponds to position 955 of SEQ ID NO: 32). Advantageously, the at least one mutation is introduced by genetic engineering techniques. In an embodiment, the mutation is introduced by targeted mutagenesis. One particularly promising targeted mutagenesis method is genome editing using engineered nucleases, wherein a site-specific double-strand break is induced in the genomic DNA of a target cell using an engineered nuclease. Engineered nucleases useful in genome editing methods include meganucleases, zinc finger nucleases (ZFNs), transcription activator-like effector-based nucleases (TALEN), and clustered regularly interspaced short palindromic repeats (CRISPR)-associated nucleases. Particularly useful genome editing methods include CRISPR/Cas9-based targeted mutagenesis methods and CRISPR/Cpf1-based targeted mutagenesis methods; see e.g. Brooks et al. (2014) Plant Physiol 166, 1292-1297.

In another embodiment, the mutation is introduced by random mutagenesis. Genetic engineering techniques to introduce a random mutation are well known by the person skilled in the art and include ionizing radiation (X-ray, Gamma rays and neutrons); chemical mutagenesis. The random mutagenesis may be for example performed by Ethyl Methane sulfonate (EMS) treatment.

In another embodiment, the mutation is introduced by TILLING.

In an alternative embodiment, the at least one genetic alteration that increases activity of PDF2 comprises or consists in the overexpression of PDF2.

Thus, an aspect of the invention relates to a method of producing a plant with an increased density of glandular trichomes comprising a step of overexpressing PDF2 having the amino acid sequence SEQ ID NO: 1 or an amino acid sequence having at least 70%, at least 75%, at least 80%, at least 85%, at least 90%, at least 95%, at least 96%, at least 97%, at least 98% or at least 99% identity with SEQ ID NO: 1.

Preferably the overexpression of PDF2 is carried out by the introduction of genetic alteration(s) in the promoter of *PDF2.*

The expression of a gene is increased by a promoter, particularly in a plant or part thereof, if as a result of said expression an altered spatial distribution and/or an increased quantity of the gene product is found, compared to the expression of the wild-type (regular) promoter for this gene in, for example, a non-transgenic plant or part thereof. Preferred levels of the increase of expression lead to a more than 2-fold, more than 5-fold, more than 10- fold or even more than 20-fold higher expression of the gene, when compared to the expression of the wild-type (regular) promoter for this gene. Increase of expression or activity can be measured using well-known methods in the art, such as mRNA detection (e.g. using rtPCR), promoter activity assays, and Western blotting (immunoblot) analysis. Any suitable ubiquitous or tissue-specific plant promoter can be used, as long as the promoter causes an overexpression of the gene. Respective promoters are known to the person of skill, and described in the state of the art and selected from ubiquitous and tissue-specific plant promoters, such as BSV, CaMV 35S, TA29, actin, FBP7, CMPS, Lhca3.St.I, Mannopin synthase, RolD, Uepl, Ubiquitin and NapA, Gtl (synonymously termed GluA-2), 2S, CuMFTI, Dc3, HaG3-A, LeB4, LegA, Phas, Psl, Str, and USP.

The method according to the first aspect of the invention may comprise a step of selecting a plant with an increased density of glandular trichomes and/or an increased production of at least one metabolite, in particular further to a step of introducing a mutation by random mutagenesis.

The increased density of glandular trichomes and/or an increased production of at least one metabolite is evaluated by comparison with a control plant. As defined above, a control plant is of the same genetic background and is growing in the same environment as the described plant, but it does not comprise the at least one genetic alteration according to the invention, while the described plant does comprise the at least one genetic alteration according to the invention. Typically, the control plant may be the wild type plant.

The step of selecting a plant with an increased density of glandular trichomes may be carried out by evaluating with a scanning electron microscope and/or with a visual evaluation of the plant surface. This increase density of glandular trichomes is of at least 10%, at least 20%, at least 30%, at least 40%,at least 50%, at least 60%, at least 70%, at least 80%, at least 90% or at least 100%.

The step of selecting a plant with an increased production of at least one metabolite may be carried out by GC-MS chromatography. This increase in the production of metabolites is of at least 10%, at least 20%, at least 30%, at least 40%,at least 50%, at least 60%, at least 70%, at least 80%, at least 90% or at least 100%.

The metabolite produced by the glandular trichome is dependent on the plant.

The Table 2 below shows some examples of metabolites produced by the glandular trichome of various plants.

**Table 2**

| **Plant** | **Metabolite** |
|---|---|
| *Salvia sclarea* (clary sage) | sclareol, lilanyl acetate, manool |
| *Salvia officinalis* | tricyclic diterpenoids, ferruginol, 11-hydroxylferruginol, sugiol, 11-hydroxylsugiol, carnosic acid, carnosol, rosmanol, and methyl carnosic acid, tanshinone |
| *Salvia rosmarinus* | carnosic acid , carnosol, rosmarinic acid, ursolic acid, alpha-pinene, camphor, camphene |
| *Salvia splendens* (scarlet sage) | caffeic acid, rosmarinic acid, cosmosiin and cinaroside, luteolin, apigenin, coumarin, dihydroxycoumarin |
| *Salvia hispanica* (chia) | polyunsaturated fatty acids (PUFA), apigenin and luteolin glycosides |
| *Perilla frutescens* (Korean perilla) | caffeic acid, rosmarinic acid, luteolin, apigenin, linoleic acid, palmitic acid, oleic acid |
| *Thymus quinquecostatus* | thymol, carvacrol, citral, geraniol, and nerolidol |
| *Mentha longifolia* (horse mint) | menthol, menthone, pulegone, 1,8-cineole |
| *Artemisia annua* (sweet wormwood) | artemisinin |
| *Solanum lycopersicum* (tomato) | acylsugars, sesquiterpenes, diterpene, flavonoids |
| *Solanum tuberosum* (potato) | sesquiterpenes, polyphenols |
| *Capsicum annuum* (red pepper) | terpenoids, polyphenols |
| *Nicotiana tabacum* (tobacco) | capsidiol, cembratriendiol |
| *Cucumis melo* (melon) | polyphenols, flavonoids |
| *Cucumis sativus* (cucumber) | silicon |
| *Cannabis sativa* (cannabis) | cannabinoids |

In an embodiment, the plant is selected from the group consisting of *Lamiaceae, Asteraceae, Solanaceae, Cucurbitaceae, Malvaceae* and *Cannabaceae.*

In an embodiment, the plant is selected from the group consisting of *Salvia, Cannabis, Artemisia, Perilla, Thymus, Mentha, Solanum, Capsicum, Cucumis, Gossypium* and *Nicotiana.* In an embodiment, the plant is selected from the group consisting of *Salvia sclarea, Cannabis sativa, Artemisia annua, Salvia splendens, Salvia hispanica, Salvia officinalis, Salvia rosmarinus, Perilla frutescens, Thymus, Mentha longifolia, Mentha piperita, Mentha spicata, Mentha arvensis, Solanum lycopersicum, Solanum tuberosum, Capsicum annuum, Cucumis melo, Cucumis sativus, Gossypium hirsutum, Nicotiana tabacum* and *Nicotiona glutinosa.*

Preferably, the plant is Lamiaceae, more preferably *Salvia,* most preferably *Salvia sclarea.*

In an embodiment, the metabolite is selected from the group consisting of acylsugar, fatty acid, terpenoid, terpene, alkaloid, polyphenol, cannabinoid, tanshinone and coumarin and mixtures thereof. Fatty acid may be selected from the group consisting of linoleic acid, palmitic acid, oleic acid and polyunsaturated fatty acids. Terpenoid may be selected from the group consisting of manool, camphor, Menthol, 1,8 cineole, geraniol, cembratriendiol and capsidiol. Terpene may be selected from the group consisting of sclareol, carnosic acid, carnosol, rosmanol, methyl carnosic acid, ursolic acid, alpha-pinene, camphene, thymol, carvacrol, menthone, pulegone, citral, nerolidol, artemisinin and lilanyl acetate. Polyphenol may be flavonoid or non-flavonoid polyphenol. Flavonoid polyphenol may be selected from the group consisting of cosmosiin, cinaroside, luteolin and apigenin. Non flavonoid polyphenol may selected from the group consisting of ferruginol, 11-hydroxylferruginol, sugiol, 11-hydroxylsugiol, rosmarinic acid, caffeic acid.

Preferably, the metabolite is terpene or terpenoid, more preferably the metabolite is sclareol. In a second aspect, the present invention relates to a method of producing a plant with an increased production of at least one metabolite.

Indeed, the amount of metabolites produced by a plant is often tightly correlated to the density of glandular trichomes present at the surface of its epidermis. Increasing glandular trichome density is therefore a new plant breeding strategy to enhance the yield in metabolites of interest.

Advantageously, the plant having an increased density of glandular trichomes, also shows an increase of at least one metabolite.

Therefore, the method of producing a plant with an increased production of at least one metabolite may comprise a step of producing a plant with an increased density of glandular trichomes according to the first aspect of the invention.

Typically, the method of producing a plant with an increased production of at least one metabolite comprises a step of introducing at least one genetic alteration that increases activity of PDF2 of said plant, PDF2 having the amino acid sequence SEQ ID NO: 1 or an amino acid sequence having at least 70%, at least 75%, at least 80%, at least 85%, at least 90%, at least 95%, at least 96%, at least 97%, at least 98% or at least 99% identity with SEQ ID NO: 1.

Advantageously, said at least one genetic alteration results in an increased production of at least one metabolite.

The at least one genetic alteration is as defined in the first aspect of the invention.

Thus, according to various embodiments of the invention, the at least one genetic alteration comprises or consists in a mutation:
- in the nucleic acid sequence that encodes the amino acids 58-117 of the START domain, and/or
- that results in a substitution, with a S or T, preferably with S, at position which corresponds to the position 111 of the amino acid sequence SEQ ID NO: 16, and/or
- selected from the group consisting of a substitution with a nucleotide T at a position which corresponds to position 3 209 of SEQ ID NO: 32, a substitution with a nucleotide A at a position which corresponds to position 3 209 of SEQ ID NO: 32, a substitution with a nucleotide A at a position which corresponds to position 3 210 of SEQ ID NO: 32 and a substitution with a nucleotide A at a position which corresponds to position 3 210 of SEQ ID NO: 32 and with a T or C at a position which corresponds to position 3 210 of SEQ ID NO: 32, and/or
- that results in a substitution, with a S or a T, preferably with S, at position which corresponds to position 347 of the amino acid sequence SEQ ID NO: 1.
- that results in a substitution, with a M or C, preferably with M, at the position 83 of the START domain of the PDF2 protein having the amino acid sequence SEQ ID NO: 16, and/or
- selected from the group consisting of a substitution with a nucleotide a substitution with a nucleotide A at a position which corresponds to position 3 042 of SEQ ID NO: 32, a substitution with a nucleotide G at a position which corresponds to position 3 043 of SEQ ID NO: 32 and with a nucleotide T at a position which corresponds to position 3 044 of SEQ ID NO: 32, a substitution with a nucleotide G at a position which corresponds to position 3 043 of SEQ ID NO: 32 and with a nucleotide C at a position which corresponds to position 3 044 of SEQ ID NO: 32, and/or
- that results in a substitution, with a M or C, preferably with M, at position which corresponds to position L319 of the amino acid sequence SEQ ID NO: 1.

According to various alternative embodiments of the invention, the at least one genetic alteration comprises or consists in:
- the overexpression of PDF2,
- preferably the overexpression of PDF2 being carried out by the introduction of genetic alteration(s) in the promoter of *PDF2.*

As for the first aspect, the method according to the second aspect of the invention may comprise a step of selecting a plant with an increased density of glandular trichomes and/or an increased production of at least one metabolite.

The plants and the metabolites are also those defined according to the first aspect of the invention.

In a third aspect of the invention, the present invention relates to a method for identifying a plant having an increased density of glandular trichomes and/or an increased production of at least one metabolite comprising the step of the detecting in the plant at least one genetic alteration, preferably at least one mutation, in a *PDF2* gene of said plant, the at least one alteration being as defined in the first and/or second aspect of the invention, thereby identifying a plant having an increased density of glandular trichomes and/or an increased production of at least one metabolite.

Methods of detection are well-known in the art and include PCR based methods, sequencing methods, and hybridization methods, in which primers or are used.

In an embodiment, a method for identifying a plant having an increased density of glandular trichomes and/or an increased production of at least one metabolite may comprise the steps of:
- providing a population of plant,
- screening the population of plant for a member comprising the at least one mutation as defined in the first and/or second aspect of the invention,
- selecting member of the population, wherein said member comprises said at least one mutation.

The present invention also encompasses a kit for identifying a plant having an increased density of glandular trichomes and/or an increased production of at least one metabolite, wherein said kit comprises an oligonucleotide primer pair for amplifying a region of a *PDF2* gene, preferably of the region encoding the START domain, wherein the at least one mutation being as defined in the first and second aspect of the invention is comprised. The oligonucleotide primer pair may comprise for example the forward primer having the nucleic acid sequence TGAGATGGACCTGATTAGAGACGAGG (SEQ ID NO:42) or CTGGATATGGCGCACAAAACACC(SEQ ID NO:43) and the reverse primer having the nucleic acid sequence GTGCATTTGATGAGGCTATTGACGG (SEQ ID NO: 44) or TAGTATTTGAAAGGCCACCGTGAGC (SEQ ID NO: 45).

### Plant

In a fourth aspect of the invention, the present invention relates to a genetically engineered plant obtainable, preferably obtained, by the methods according to the first and/or second aspect of the invention, wherein said plant has an increased density of glandular trichomes and/or an increased production of at least one metabolite compared to a control plant. Advantageously, the genetically engineered plant, comprising at least one genetic alteration that increases activity of PDF2, wherein said plant has an increased density of glandular trichomes and/or an increased production of at least one metabolite compared to a control plant. The at least one genetic alteration being as defined in the first and/or second aspect of the invention.

The plant according to the present invention is obtained by genetic engineering techniques, that is to say that plant is not exclusively obtained by an essentially biological process.

In an embodiment, the plant is selected from the group consisting of *Lamiaceae, Asteraceae, Solanaceae, Cucurbitaceae, Malvaceae* and *Cannabaceae.*

In an embodiment, the plant is selected from the group consisting of *Salvia, Cannabis, Artemisia, Perilla, Thymus, Mentha, Solanum, Capsicum, Cucumis, Gossypium* and *Nicotiana.* In an embodiment, the plant is selected from the group consisting of *Salvia sclarea, Cannabis sativa, Artemisia annua, Salvia splendens, Salvia hispanica, Salvia officinalis, Salvia rosmarinus, Perilla frutescens, Thymus quinquecostatus, Mentha longifolia, Mentha piperita, Mentha spicata, Mentha arvensis, Solanum lycopersicum, Solanum tuberosum, Capsicum annuum, Cucumis melo, Cucumis sativus, Gossypium hirsutum, Nicotiana tabacum* and *Nicotiona glutinosa.*

Preferably, the plant is Lamiaceae, more preferably *Salvia,* most preferably *Salvia sclarea.*

A further aspect of the invention encompasses a seed that produces the plant of the invention. It is a further embodiment to provide a plant part, organ or tissue obtainable from a plant of the invention or any other part of the plant, including but not limited to leaves, stems, petals, petioles, peduncles and seeds, which presents glandular trichomes and produce metabolites. The present invention also relates to the use of the plant of the invention for producing at least one metabolite. In an embodiment, the metabolite is selected from the group consisting of acylsugar, fatty acid, terpenoid, terpene, alkaloid, polyphenol, cannabinoid, tanshinone and coumarin and mixtures thereof as defined above.

### FIGURES

Figure 1 is a schematic representation of the region encoding the START domain of PDF2 gene of the *Salvia sclarea* wild type and with the mutations A347S, L319Mand R335*.
Figure 2 shows the trichome density of wild type clary sage (WT) and *Sspdf2* mutant of *Salvia sclarea* . LC : long capitate glandular trichome ; SC: short capitate glandular trichome ; P: peltate glandular trichome; T: non glandular trichome.
Figure 3 shows the production of sclareol of wild type clary sage (WT) and *Sspdf2_A347S* mutant of *Salvia sclarea* . Sclareol content expressed as a percentage of calyx dry weight. Bars represent the mean ± SD (*n*=5).
Figure 4 shows the production of linalyl acetate of wild type clary sage (WT) and *Sspdf2_A347S* mutant of *Salvia sclarea.* Lilanyl acetate content expressed as a percentage of calyx dry weight. Bars represent the mean ± SD (n=5).
Figure 5 shows the production of sclareol of wild type clary sage (WT) and *Sspdf2_L319M* mutant of *Salvia sclarea* . Sclareol content expressed as a percentage of calyx dry weight. Bars represent the mean ± SD (n=5).
Figure 6 shows the production of linalyl acetate of wild type clary sage (WT) and *Sspdf2_L319M* mutant of *Salvia sclarea* . Lilanyl acetate content expressed as a percentage of calyx dry weight. Bars represent the mean ± SD (n=5).
Figure 7 shows the production of sclareol of wild type clary sage (WT) and *Sspdf2_R335** mutant of *Salvia sclarea* . Sclareol content expressed as a percentage of calyx dry weight. Bars represent the mean ± SD (n=5).
Figure 8 shows the production of linalyl acetate of wild type clary sage (WT) and *Sspdf2_R335** mutant of *Salvia sclarea* . Lilanyl acetate content expressed as a percentage of calyx dry weight. Bars represent the mean ± SD (*n*=5).

### Examples

### Material and methods

### Plant material and EMS mutagenesis

*Salvia sclarea* seeds were originated from the National Conservatory of Perfume Plants (CNPMAI).

To set-up the 'kill-curve' analysis, batches of 200 seeds were immersed in a solution of different EMS concentrations (0.5, 1 and 1.5%) for 16 h at room temperature with stirring. Seeds were washed 2 times with Na2S2O3 1M (Sigma-Aldrich) for 5 min each and 6 times with tap water for 5 min each and finally for 30 min. 80 to 100 seeds were then transferred to wet tissue papers in Petri dishes in a growth chamber at 22°C with 8h of photoperiod.

The optimal concentrations (0.5% and 0.75%) were used to mutagenize a batch of ~10,000 seeds. Treated seeds were sown on pots and grown in a tunnel. 4,795 were planted as seedlings in field. Between May and June next year, when the first flower buds emerged, micro-perforated plastic bags were placed on the stems to self-pollinate the plants. 4,254 M2 seeds patches were then harvested independently and stored in cold room (4°C with 30% RH).

### Genomic DNA extraction

Batches of eight seeds per wild-type and M2 families were grown in pots for two weeks in growth chamber (16 h of day; 27 °C during the day, 21 °C at night; 60% hygrometry). Eight leaf discs (diameter 10mm) were collected with a cookie-cutter in 96 well-plates containing two steel beads (4mm) per well. Tissues were lyophilised and disrupted using a Tissuelyzer II (Qiagen) two times for 30 sec at 25Hz. Genomic DNA was extracted using the Dneasy 96 Plant kit (Qiagen, Hilden, Germany). To check the quality and the uniformity of genomic DNA, samples were systematically run on a 1% agarose gel. The quantity and purity of genomic DNA were measured by a NanoDropTM 2000 Spectrophotometer (Thermo Scientific, USA). Finally, eight fold pooling plates of DNA were prepared. A population of 4242 arrayed DNAs from mutagenized individuals was stored at -20°C.

### Mutation detection by TILLING-NGS

The sequencing of amplicons was done by Next-Generation Sequencing (NGS) technology (method unpublished) to detect putative point mutations. DNA libraries from 4,242 mutated lines were constructed for each PCR fragment. MiSeq runs were performed with the MiSeq V3-kit (600 cycles) and 2 × 300 cycles' pair-end sequencing was carried out. Raw data were analysed by SENTINEL (Inter Deposit Digital Number: FR001.240004.000.R.P.2016.000.10000) a bioinformatics pipeline developed in our laboratory to detect rare SNPs. Prediction of the impact of mismatch on protein function was made using the SIFT program (sift.jcvi.org/).

### Genotyping and Phenotyping mutants

In order to isolate homozygous mutants, 20 to 50 seeds of identified M2 families were sown in pots and maintained 2 weeks in growth chamber (16 h of day; 27 °C during the day, 21 °C at night; 60% hygrometry). We performed a DNA extraction using Dneasy 96 Plant kit (Qiagen, Hilden, Germany), an amplification of the mutated sequence by PCR and a Sanger sequencing (Eurofins) on individual plantlets. Pots were moved outdoor at the end of autumn for natural vernalization and growing until flowering.

### Sclareol and linalyl acetate quantification by GC-MS

Mature clary sage calyces were collected just after the fall of the petals from wild-type and Ssdxs2-1 and Ssdxs2-2 mutants. Calyces were immersed in hexane (1.5 mL per calyx) without grinding to extract metabolites present at the surface. 10-undecen-1-ol (0.75 mM) was employed as internal standard and was added in the solvent before extraction. After 2 h of agitation, the extract was centrifuged at 13,000 rpm for 10 min to eliminate potential dust. Extracts were directly analysed using a 7890B/5977A GC-MS system from Agilent, according to a protocol adapted from Laville et al. ("Amphilectane diterpenes from Salvia sclarea: Biosynthetic considerations", J. Nat. Prod., 2012, 75: 121-126). The system was equipped with a 10 m guard column and an Rxi-5Sil MS column (length, 30 m; inner diameter, 0.25 mm; film thickness, 0.25 µm) (Restek, Bellefonte, PA, USA). One µl of sample was injected with a split ratio of 30:1. Oven temperature was set to 110 °C for 2 min, then increased to 270 °C at a rate of 10 °C/min, and finally set to 270 °C for 2 min. Other temperatures were set as follows: injector, 270 °C; transfer line, 270 °C; source, 230 °C; quadrupole, 150 °C. The carrier gas was helium at a constant flow of 1 mL/min. The quadrupole mass spectrometer was switched on after a solvent delay of 5 min and was programmed to scan from 35 u to 350 u. Data analysis was carried out using the MassHunter Quantitative Analysis software from Agilent. Sclareol and linalyl acetate were identified by comparison with analytical standards purchased from Sigma-Aldrich. Absolute quantification of sclareol and linalyl acetate was performed using a calibration curve prepared with analytical standards.

### Scanning electron microscopy and trichome numbering

Leaves (around 4cm long) were collected from 8 months wild-type plants and wo-1 mutants. Freshly cut leaves were introduced in the observation chamber of a scanning electron microscope (SEM, Helios NanoLab 660 instrument, FEI) without metallization or any other sample preparation. Observation of calyx abaxial face (outer face) was performed with the voltage set to 0.35 kV.

For trichome numbering and length measure, portions of 1.70 mm × 2.45 mm of the abaxial face were scanned at low pressure. The Cell Count Plugin of ImageJ (http://rsb.info.nih.gov/ij) was used to calculate trichome density.

### Results

The results for three *Salvia sclarea* mutants are discussed here.

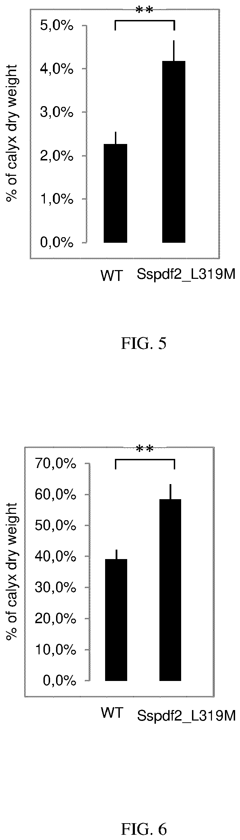

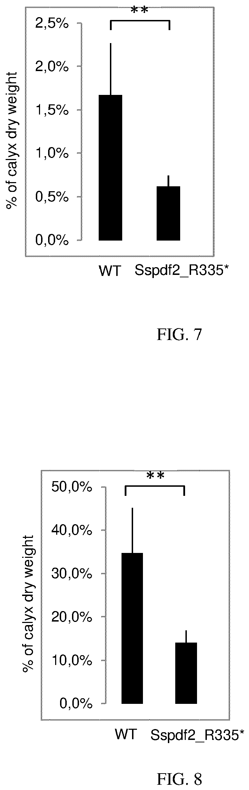

The Inventors have shown that the mutant stop: *Sspdf2_R335** results in drastically reduced production of sclareol and lilanyl acetate (figures 6 and 7). This mutant shows the important role of PDF2 expression in the development of glandular trichomes.

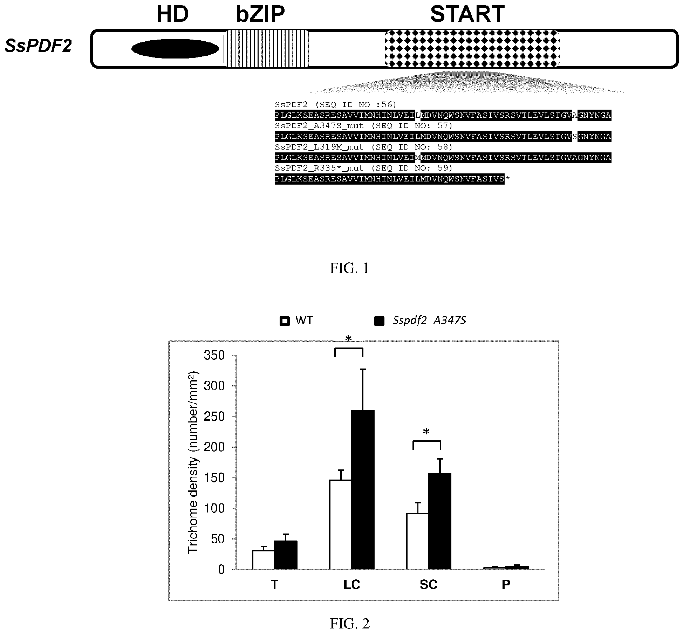

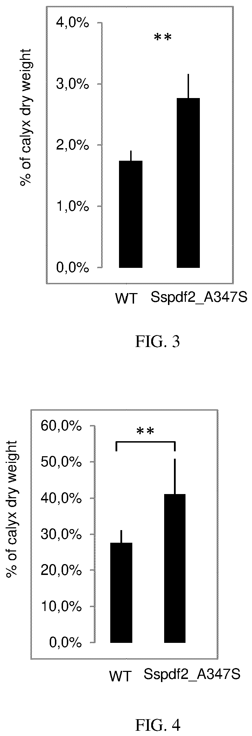

The *Sspdf_A347S* mutant is obtained by a A347S substitution in PDF2. The Inventors have observed that the trichome density of the *Sspdf_A347S* mutant is higher than the one of the wild type plant (figure 2). The increase is almost 50% for each type of glandular trichomes. An increased production of sclareol (figure 3) and lilanyl acetate (figure 4) by the *Sspdf_A347S* mutant compared to the wild type is associated with the increased density of glandular trichomes.

The Inventors have identified a second gain of function mutant in *S*. *sclarea:* the *Sspdf2_L319M* mutant. This mutant have been obtained by a L319M substitution in PDF2. From the figures 5 and 6, the Inventors demonstrated that the production of sclareol and lilanyl acetate respectively is increased for the *Sspdf2_L319M* mutant compared to the wild type plant.

### REFERENCES

Camille Chalvin, Stéphanie Drevensek, Michel Dron, Abdelhafid Bendahmane, Adnane Boualem "Genetic control of glandular trichome development", Trends Plant Sci. 2020 May;25(5):477-487. doi: 10.1016/j.tplants.2019.12.025. Epub 2020 Jan 23.
Lange Bernd Markus, "The evolution of plant secretory structures and emergence of terpenoid chemical diversity", Annu Rev Plant Biol. 2015:66:139-59. doi: 10.1146/annurev-arplant-043014-114639. Epub 2015 Jan 19.
Fahn, A. (2000) Structure and Function of Secretory Cells. In: Hallahan, D.L., Gray, J.C. and Callow, J.A., Eds., Advances in Botanical Research, Incorporating Advances in Plant Pathology, Volume 31, Plant Trichomes, Academic Press, London, 37-66. http://dx.doi.org/10.1016/S0065-2296(00)31006-0
Figueiredo, A.S.T., Resende, J.T.V., Morales, R.G.F. et al. The role of glandular and non-glandular trichomes in the negative interactions between strawberry cultivars and spider mite. Arthropod-Plant Interactions 7, 53-58 (2013). https://doi.org/10.1007/s11829-012-9218-z
B J Glover, "Differentiation in plant epidermal cells", J Exp Bot. 2000 Mar;51(344):497-505. doi: 10.1093/jexbot/51.344.497.
Alexandre Huchelmann, Marc Boutry, Charles Hachez "Plant GlandularTrichomes: Natural Cell Factories of High Biotechnological Interest", Plant Physiol. 2017 Sep;175(1):6-22. doi: 10.1104/pp.17.00727. Epub 2017 Jul 19.
Tetsuya Ishida 1, Tetsuya Kurata, Kiyotaka Okada, Takuji Wada, "A genetic regulatory network in the development of trichomes and root hairs" Annu Rev Plant Biol. 2008:59:365-86. doi: 10.1146/annurev.arplant.59.032607.092949.
G Karabourniotis, G Kofidis, C Fasseas, V Liakoura, I Drossopoulos, "Polyphenol deposition in leaf hairs of Olea europaea (Oleaceae) and Quercus ilex (Fagaceae)", Am J Bot. 1998 Jul;85(7):1007.
SJ Lolle 1, RE Pruitt, "Epidermal cell interactions: a case for local talk", Trends Plant Sci. 1999 Jan;4(1):14-20. doi: 10.1016/s1360-1385(98)01353-3.
Sitakanta Pattanaik, Barunava Patra, Sanjay Kumar Singh, Ling Yuan, "An overview of the gene regulatory network controlling trichome development in the model plant, Arabidopsis", Front Plant Sci. 2014 Jun 5:5:259. doi: 10.3389/fpls.2014.00259. eCollection 2014.
Laura Serna, Cathie Martin, "Trichomes: different regulatory networks lead to convergent structures", Trends Plant Sci. 2006 Jun;11(6):274-80. doi: 10.1016/j.tplants.2006.04.008. Epub 2006 May 11.
Yan A, Pan J, An L, Gan Y, Feng H. The responses of trichome mutants to enhanced ultraviolet-B radiation in Arabidopsis thaliana. J Photochem Photobiol B. 2012:29-35

## Claims

1. A method of producing a plant with an increased density of glandular trichomes comprising: a step of introducing at least one genetic alteration that increases activity of PRODERMAL FACTOR2 (PDF2) of said plant, wherein PDF2 preferably has an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39 or an amino acid sequence having at least 70%, preferably at least 75 %, moreover preferably at least 80 %, moreover preferably at least 85 %, moreover preferred at least 90 % and especially preferred at least 99 % identity with SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

2. A method of producing a plant with an increased production of at least one metabolite comprising: a step of introducing at least one genetic alteration that increases activity of PDF2 of said plant, wherein PDF2 preferably has an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39 or an amino acid sequence having at least 70%, preferably at least 75 %, moreover preferably at least 80 %, moreover preferably at least 85 %, moreover preferred at least 90 % and especially preferred at least 99 % identity with SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 38 and SEQ ID NO: 39.

3. The method according to claim 1 or 2, wherein PDF2 has an amino acid sequence according to SEQ ID NO: 1 or an amino acid sequence having at least 70 %, preferably at least 75 %, moreover preferably at least 80 %, moreover preferably at least 85 %, moreover preferred at least 90 % and especially preferred at least 99 % identity with an amino acid sequence according to SEQ ID NO.: 1.

4. The method according to any one of claims 1 to 3, wherein the at least one genetic alteration that increases activity of PDF2 comprises at least one mutation in the gene encoding PDF2.

5. The method according to any one of claims 1 to 4, wherein the at least one mutation comprises a mutation in the region encoding the *StAR-related lipid-transfer* (*START*) domain of PDF2, the START domain having an amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 40 and SEQ ID NO: 41 or an amino acid sequence having at least 70%, preferably at least 75 %, moreover preferably at least 80 %, moreover preferably at least 85 % and especially preferred at least 90 % identity with an amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 40 and SEQ ID NO: 41.

6. The method according to claim 5, wherein the START domain has an amino acid sequence selected from the group consisting of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 40 and SEQ ID NO: 41, preferably wherein the START domain has an amino acid sequence according to SEQ ID NO.: 16.

7. The method according to any one of claims 4 to 6, wherein the at least one mutation comprises:
- a mutation that results in a substitution with a S, T, N or Q, preferably a S, at a position which corresponds to the position A347 of PDF2 having the amino acid sequence SEQ ID NO: 1 and/or
- a mutation that results in a substitution with a M or C, preferably a M, at a position which corresponds to the position L319 of PDF2 having the amino acid sequence SEQ ID NO: 1.

8. The method according to any one of claims 1 to 3, wherein the at least one genetic alteration that increases activity of PDF2 comprises the overexpression of PDF2, preferably the overexpression of PDF2 due to the introduction of genetic alteration(s) in the promoter of *PDF2.*

9. The method according to any one of claims 1 to 8, wherein the method further comprises a step of selecting a plant with an increased density of glandular trichomes and/or an increased production of at least one metabolite, preferably wherein the method is not an essentially biological process.

10. The method according to any one of claims 1 to 9, wherein the density of glandular trichomes is increased of at least 10% compared to a control plant.

11. The method according to any one of claims 2 to 9, wherein the production of at least one metabolite is increased of at least 10 %, compared to a control plant.

12. The method according to any claims 2 to 11, **characterized in that** the metabolite is selected from the group consisting of acylsugar, fatty acid, terpenoid, terpene, alkaloid, polyphenol, cannabinoid, tanshinone and coumarin and mixtures thereof.

13. A genetically engineered plant obtained or obtainable by the method according to any one of claims 1-12, wherein said plant has an increased density of glandular trichomes and/or an increased production of at least one metabolite compared to a control plant.

14. The method according to any one of claims 1 to 12 or the plant according to claim 13, wherein the plant is selected from the group consisting of the family *Lamiaceae, Asteraceae, Solanaceae, Cucurbitaceae, Malvaceae* and *Cannabaceae.*

15. Use of the genetically engineered plant of claim 13 or 14 or a plant obtained by the method according to any one of claims 1-12 for producing at least one metabolite.

16. A kit for identifying a plant having an increased density of glandular trichomes and/or an increased production of at least one metabolite, wherein said kit comprises an oligonucleotide primer pair for amplifying a region of the *PDF2* gene, preferably of the region encoding the START domain, wherein the at least one mutation in the *PDF2* gene as defined in any one of claims 4-7 is detected with the oligonucleotides.

17. A method for identifying a plant having an increased density of glandular trichomes and/or an increased production of at least one metabolite comprising the step of the detecting the at least one genetic alteration as defined in any one of claims 1-12 in the plant, preferably the at least one mutation as defined in any one of claims 4-7.
